# EUROPEAN PATENT SPECIFICATION

(11) **EP 2 970 043 B1**
(45) Date of publication and mention of the grant of the patent: **09.10.2019**
(21) Application number: 14712476.2
(22) Date of filing: 05.03.2014
(51) Int. Cl.: B01J 29/70, C10G 69/12, C10G 50/00, B01J 29/40, C07C 2/12, C07C 5/05, C10L 1/04

(54) **PRODUCTION OF LUBRICANT BASE OILS FROM DILUTE ETHYLENE FEEDS**
ERZEUGUNG VON SCHMIERBASISÖLEN AUS VERDÜNNTEN ETHYLENEINSPEISUNGEN
PRODUCTION D'HUILES DE BASE LUBRIFIANTES À PARTIR D'ALIMENTATIONS D'ÉTHYLÈNE DILUÉES

(30) Priority: 15.03.2013 US 201361789051 P
(43) Date of publication of application: 20.01.2016
(73) Proprietor: ExxonMobil Research and Engineering Company, Annandale, NJ 08801-0900 (US)
(72) Inventor: DAAGE, Michel, Hellertown, PA 18055 (US); BROWN, Stephen, H., LEBANON, NJ 08833 (US); SANCHEZ, Eugenio, Pitman, NJ 08071 (US); BHORE, Nazeer, A., 07920 New Jersey (US); WELCH, Robert, Charles William, Baton Rouge, LA 70808 (US); HOLTZER, Gretchen, L., GRAND FORKS AFB, North Dakota 58204 (US); ANDREWS, Arthur T., Phildelphia, PA 19103 (US)
(74) Representative: ExxonMobil Chemical Europe Inc.
(86) International application number: PCT/US2014/020592
(87) International publication number: WO 2014/149731

(56) References cited:
- US-A- 4 618 737
- US-A- 4 754 096
- US-A- 4 822 477
- US-A- 5 202 015
- US-A1- 2002 117 424
- US-A1- 2003 171 632
- US-A1- 2011 243 797

## Description

### FIELD

Systems and methods are provided for production of lubricant oil basestocks by oligomerization of a dilute ethylene feedstock.

### BACKGROUND

Oligomerization/polymerization of ethylene is a well known technology for producing alpha olefins and polyethylene. However, the technology typically requires a complex purification scheme to recover "polymer grade" ethylene from sources such as steam cracker or light gas recovery from a fluid catalytic cracker (FCC). Up to two-thirds of the cost of manufacturing "polymer grade" ethylene can be associated with such purificaiotn of an ethylene stream prior to the actual oligomerization reaction. The purification can be required, in part, to avoid "poisoning" of the oligomerization catalyst with common heteroatom species present in a FCC light gas recovery stream (or other refinery stream), such as mercaptans and/or hydrogen sulfide.

U.S. Patent Application Publication 2010/0249474 describes a process for oligomerizing dilute olefins. A dilute ethylene stream, such as an FCC dry gas stream, can be used as the feed for oligomerization. A catalyst corresponding to an amorphous silica-alumina base with supported Group VIII and/or Group VIB metals can be used to catalyze the oligomerization. Such a catalyst is described as being resistant to feed impurities such as hydrogen sulfide, carbon oxides, and ammonia. The catalyst is described as having a silica to alumina ratio of no more than 30, such as no more than 20. In the description of the catalyst, it is noted that the silica alumina ratio of the catlyst should not be greater than 30.

U.S. Patent Application Publication 2011/0243797 discloses apparatus for oligomerizing dilute ethylene that may be derived from an FCC product. It is disclosed that ethylene in dilute ethylene streams, such as an FCC dry gas stream, can be catalytically oligomerized with a Group VIIIB metal on a low acidity amorphous silica catalyst. U.S. Patent Number 4,618,737 discloses a two stage process for olefin oligomerization according to which a lower olefin feed is converted to distillate range hydrocarbons in the presence of an aluminosilicate zeolite such as ZSM-5. and then the distillate effluent is further polymerized to lubricant range hydrocarbons in the presence of a ditertiary alkylperoxide catalyst. It is disclosed that typical feedstocks consist essentially of C3-C6 mono-olefins.

### SUMMARY

In an aspect, a method for forming fuel and lubricant products is provided. The method includes comprising exposing a feedstock comprising 5 to 50 vol% of ethylene to a catalyst comprising a zeolite with 10-member rings under effective oligomerization conditions to produce an oligomerized effluent, wherein the feedstock consist in an off-gas from a fluid catalytic cracking process, wherein the off-gas comprises H₂S, organic sulfur, and/or other sulfur compounds, and wherein the oligomerized effluent comprises organic sulfur in an amount of at least 25 wppm; hydroprocessing at least a portion of the oligomerized effluent under effective hydroprocessing conditions to form a hydroprocessed effluent, wherein hydroprocessing at least a portion of the oligomerized effluent comprises hydrotreating at least a portion of the oligomerized effluent under hydrotreating conditions including temperatures of 200°C to 450°C, pressures of 250 psig (1.8 MPag) to 5000 psig (34.6 MPag), liquid hourly space velocities (LHSV) of 0.1 hr⁻¹ to 10 hr⁻¹, and hydrogen treat rates of 200 scf/B (35.6 m³/m³) to 10,000 scf/B (1781 m³/m³); and separating the hydroprocessed effluent to form a fraction having a boiling point of 350°F (177°C) or less, one or more lubricant base oil fractions, and at least one diesel fuel fraction having a lower boiling range than at least one lubricant base oil fraction.

In some additional aspects, exposing the feedstock comprising 5 to 50 vol% of ethylene to a catalyst comprising a zeolite with 10-member rings under effective oligomerization conditions to produce an oligomerized effluent can include exposing the feedstock to a first catalyst comprising a zeolite with 10-member rings under effective gas phase oligomerization conditions to form an intermediate oligomerized product; separating the intermediate oligomerized product to form at least a gas phase intermediate product and an intermediate product with an initial boiling point of at least 60°C; and exposing at least a portion of the intermediate product with a boiling point of at least 60°C to a second catalyst comprising a zeolite with 10-member rings under effective liquid phase oligomerization conditions to form the oligomerized effluent.

### BRIEF DESCRIPTION OF THE DRAWINGS

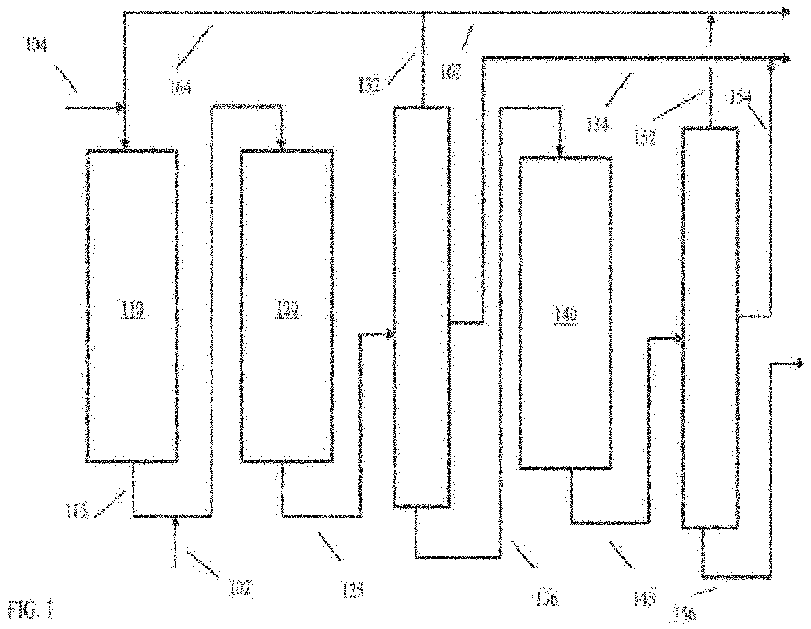

FIG. 1 schematically shows an example of a configuration suitable for forming oligomerizing an ethylene feedstock to form fuel and lubricant products.

### DETAILED DESCRIPTION

All numerical values within the detailed description and the claims herein are modified by "about" or "approximately" the indicated value, and take into account experimental error and variations that would be expected by a person having ordinary skill in the art.

### Overview

In various embodiments, methods are provided for oligomerizing a dilute ethylene feed to form oligomers suitable for use as fuels and/or lubricant base oils. The fuels and/or lubricant base oils are formed by oligomerization of impure dilute ethylene with a zeolitic catalyst, where the zeolitic catalyst is resistant to the presence of poisons such as sulfur and nitrogen in the ethylene feed. The oligomers can also be formed in presence of diluents such as light paraffins. By using a low cost feed as an input to the oligomerization reaction, namely an off-gas from an FCC process, various embodiments described herein can provide a low cost option for producing lube range molecules, such as waxes and isoparaffins with carbon numbers higher than C26.

Dilute ethylene streams can be provided by known processes such as a steam cracker, an ethane cracker, or an FCC process. The ethylene streams produced by such methods are typically impure and often contain known poisons for catalysts, such as hydrogen sulfide and mercaptans. Furthermore, higher olefins may be present, including and not limited to propylene and butenes. Butadiene and acetylene may also be present. In addition to the presence of these olefinic species, reactive light paraffins may be present and may act as diluents.

After forming oligomer products suitable for use as feeds for fuels and/or lubricant base oil production, the oligomer products can be hydroprocessed to modify the characteristics of the products. For example, the oligomer products can be hydrotreated to remove heteroatoms (such as sulfur) that are incorporated into the oligomer products. The oligomer products can also be catalytically dewaxed and/or hydrocracked in order to improve viscosity properties, cold-flow properties, and/or other properties of the oligomer products.

Group I basestocks or base oils are defined as base oils with less than 90 wt% saturated molecules and/or at least 0.03 wt% sulfur content. Group I basestocks also have a viscosity index (VI) of at least 80 but less than 120. Group II basestocks or base oils contain at least 90 wt% saturated molecules and less than 0.03 wt% sulfur. Group II basestocks also have a viscosity index of at least 80 but less than 120. Group III basestocks or base oils contain at least 90 wt% saturated molecules and less than 0.03 wt% sulfur, with a viscosity index of at least 120. In addition to the above formal definitions, some Group I basestocks may be referred to as a Group I+ basestock, which corresponds to a Group I basestock with a VI value of 103 to 108. Some Group II basestocks may be referred to as a Group II+ basestock, which corresponds to a Group II basestock with a VI of at least 113. Some Group III basestocks may be referred to as a Group III+ basestock, which corresponds to a Group III basestock with a VI value of at least 140.

### Liquid Feedstocks and Products

One way of defining a feedstock or a product is based on the boiling range of the feed or product. In this discussion, reference will be made to feed boiling ranges for clarity, but it is understood that the boiling ranges described herein are also applicable to products.

One option for defining a boiling range is to use an initial boiling point for a feed and/or a final boiling point for a feed. Another option, which in some instances may provide a more representative description of a feed (or product), is to characterize a feed based on the amount of the feed that boils at one or more temperatures. For example, a "T5" boiling point for a feed is defined as the temperature at which 5 wt% of the feed will boil off. Similarly, a "T95" boiling point is a temperature at 95 wt% of the feed will boil.

Typical lubricant boiling range feeds (or products) include, for example, feeds with an initial boiling point of at least 650°F (343°C), or at least 700°F (371°C), or at least 750°F (399°C). Alternatively, a feed may be characterized using a T5 boiling point, such as a feed with a T5 boiling point of at least 650°F (343°C), or at least 700°F (371°C), or at least 750°F (399°C). In some aspects, the final boiling point of the feed can be at least 1100°F (593°C), such as at least 1150°F (621°C) or at least 1200°F (649°C). In other aspects, a feed may be used that does not include a large portion of molecules that would traditional be considered as vacuum distillation bottoms. For example, the feed may correspond to a vacuum gas oil feed that has already been separated from a traditional vacuum bottoms portion. Such feeds include, for example, feeds with a final boiling point of 1150°F (621°C), or 1100°F (593°C) or less, or 1050°F (566°C) or less. Alternatively, a feed may be characterized using a T95 boiling point, such as a feed with a T95 boiling point of 1150°F (621°C) or less, or 1100°F (593°C) or less, or 1050°F (566°C) or less. An example of a suitable type of feedstock is a wide cut vacuum gas oil (VGO) feed, with a T5 boiling point of at least 700°F (371°C) and a T95 boiling point of 1100°F or less.

The above feed description corresponds to a potential feed for producing lubricant base oils. In some aspects, methods are provided for producing both fuels and lubricants. Because fuels are a potentially desired product, feedstocks and/or products with lower boiling components may also be suitable. For example, a fuels feedstock or product, can have a T5 boiling point of at least 250°F (121°C), such as at least 350°F (177°C). Examples of a suitable boiling range include a boiling range of from 250°F (121°C) to 700°F (371°C), such as from 390°F (200°C) to 650°F (343°C). Additionally or alternately, the upper end of the boiling range for a fuels feed (or product) can correspond to the lower end of the boiling range for a lubricant base oil feed (or product).

### Gas Feeds

DELETED.

The ethylene source for forming fuels boiling range and lubricant boiling range oligomers consists in an off-gas from a fluid catalytic cracking process that is used for another purpose. Fluid catalytic cracking is used in a refinery to convert distillate fuel and/or lubricant boiling range streams into naphtha boiling range products. During a fluid catalytic cracking process, heavier hydrocarbon molecules are "cracked" to form lighter molecules. The process typically also generates light side products including hydrogen, carbon oxides, light ends, and water. The light ends represent a mix of low weight hydrocarbon compounds, such as methane or ethane. Hydrogen sulfide is also produced if the feed to the FCC unit includes sulfur compounds, which is the typical situation. As the desired products from an FCC unit are separated or distilled out, these light gas products are separated into an "off-gas". Because of the variety of compounds in the off-gas, and because of the low hydrogen concentration, the off-gas from an FCC reactor is conventionally viewed as a low value stream. As noted above, for a conventional oligomerization process, an FCC stream must be purified to increase the concentration of ethylene and/or remove impurities.

The off-gas from an FCC process can include a variety of hydrocarbon type components, including (but not limited to) methane, ethane, ethylene, acetylene, propane, propene, butane, and/or butene. Optionally, an initial separation can be performed to remove a majority of the C₃ and C₄ components of an FCC off-gas prior to using the stream as a feed for oligomerization. The FCC off-gas can also include H₂ and species that correspond to impurities, such as H₂S and/or NH₃. The amount of ethylene in the off-gas corresponds to 5 vol% to 50 vol% of the stream. More typically, the amount of ethylene in an off-gas can be 20 vol% or less of a feed, such as 15 vol% or less or 10 vol% or less. For an off-gas where the C₃ and C₄ components have been at least partially removed, the amount of ethylene can be 25 vol% or less of the stream, such as 20 vol% or less. Methane can correspond to another large volume component in the off-gas, with a content of 25 vol% to 55 vol%. More generally, other refinery streams having an ethylene content of 50 vol% or less, and preferably an ethylene content of 20 vol% or less, can be suitable for use as a dilute ethylene feed for oligomerization. Additionally or alternately, the amount of non-olefinic components (such as alkanes) in the dilute ethylene feed can be at least 20 vol%, such as at least 30 vol%.

### Catalysts for Oligomerization

In various embodiments, oligomerization of dilute ethylene can be performed by exposing the dilute ethylene feed to a regenerable zeolite catalyst under effective oligomerization conditions. The catalysts used comprise zeolites that have a 10-member ring in the zeolite structure, such as a 10-member ring 1-D or 3-D zeolite structure. Examples of suitable zeolites include ZSM-5, ZSM-22, ZSM-23, ZSM-48, and combinations thereof. Other examples of zeolites (or other molecular sieves) include EU-1, ZSM-35 (or ferrierite), ZSM-11, ZSM-57, NU-87, and SAPO-11. Note that a zeolite having the ZSM-23 structure with a silica to alumina ratio of from 20:1 to 40:1 can sometimes be referred to as SSZ-32.

Preferably, the dewaxing catalysts used in processes according to the disclosure are catalysts with a low ratio of silica to alumina. For example, for ZSM-48, the ratio of silica to alumina in the zeolite can be less than 200:1, such as less than 110:1, or less than 100:1, or less than 90:1, or less than 75:1. In various embodiments, the ratio of silica to alumina can be from 50:1 to 200:1, such as 60:1 to 160:1, or 70:1 to 100:1.

Optionally but preferably, the dewaxing catalyst can include a binder for the molecular sieve, such as alumina, titania, silica, silica-alumina, zirconia, or a combination thereof, for example alumina and/or titania or silica and/or zirconia and/or titania. In some optional embodiments where the binder includes silica-alumina or a combination of silica and alumina, the ratio of silica to alumina in the binder can be at least 40, such as at least 50. Alternatively, the ratio of silica to alumina in the total catalyst (zeolite plus binder) can be at least 40, such as at least 50. In still other alternative embodiments, the binder can be a binder that does not contain alumina, such as a binder composed of titania, zirconia, silica, or a combination thereof.

Optionally, the zeolite catalysts can further include a metal hydrogenation component. The metal hydrogenation component is typically a Group VI and/or a Group VIII metal. Preferably, the metal hydrogenation component is a Group VIII noble metal. Preferably, the metal hydrogenation component is Pt, Pd, or a mixture thereof. In an alternative preferred embodiment, the metal hydrogenation component can be a combination of a non-noble Group VIII metal with a Group VI metal. Suitable combinations can include Ni, Co, or Fe with Mo or W, preferably Ni with Mo or W.

The metal hydrogenation component may be added to the catalyst in any convenient manner. One technique for adding the metal hydrogenation component is by incipient wetness. For example, after combining a zeolite and a binder, the combined zeolite and binder can be extruded into catalyst particles. These catalyst particles can then be exposed to a solution containing a suitable metal precursor. Alternatively, metal can be added to the catalyst by ion exchange, where a metal precursor is added to a mixture of zeolite (or zeolite and binder) prior to extrusion.

The amount of metal in the catalyst can be at least 0.1 wt% based on catalyst, or at least 0.15 wt%, or at least 0.2 wt%, or at least 0.25 wt%, or at least 0.3 wt%, or at least 0.5 wt% based on catalyst. The amount of metal in the catalyst can be 20 wt% or less based on catalyst, or 10 wt% or less, or 5 wt% or less, or 2.5 wt% or less, or 1 wt% or less. For embodiments where the metal is Pt, Pd, another Group VIII noble metal, or a combination thereof, the amount of metal can be from 0.1 to 5 wt%, preferably from 0.1 to 2 wt%, or 0.25 to 1.8 wt%, or 0.4 to 1.5 wt%. For embodiments where the metal is a combination of a non-noble Group VIII metal with a Group VI metal, the combined amount of metal can be from 0.5 wt% to 20 wt%, or 1 wt% to 15 wt%, or 2.5 wt% to 10 wt%.

The catalysts useful in oligomerization processes according to the disclosure can also include a binder. In some embodiments, the catalysts used in oligomerization processes according to the disclosure can be formulated using a low surface area binder, where a low surface area binder represents a binder with a surface area of 100 m²/g or less, or 80 m²/g or less, or 70 m²/g or less. Optionally, a ratio of zeolite surface area to binder surface area for the catalyst can be at least 80:100 such as at least 1:1. The amount of zeolite in a catalyst formulated using a binder can be from 30 wt% zeolite to 90 wt% zeolite relative to the combined weight of binder and zeolite. Preferably, the amount of zeolite is at least 50 wt% of the combined weight of zeolite and binder, such as at least 60 wt% or from 65 wt% to 80 wt%.

### Formation of Oligomers from Dilute Ethylene Feedstock

The zeolite catalysts noted above can be used to form oligomers from a dilute ethylene feedstock. The oligomerization process can be performed as a single step process or a two step process.

In a single step process, all of the oligomerization is performed in a single stage and/or in a single reactor. The dilute ethylene feedstock is introduced into the single stage as a gas phase feed. The ethylene feed is exposed to the zeolite catalyst under effective oligomerization conditions. For example, the ethylene feed can be exposed to the zeolite catalyst at a temperature of 20°C to 300°C, such as at least 25°C or at least 50°C or at least 100°C and/or preferably 250°C or less, more preferably 225°C or less. The ethylene feed can be exposed to the catalyst at a gas hourly space velocity (based on ethylene) of 1 hr⁻¹ to 500 hr⁻¹, such as at least 5 hr⁻¹ or at least 10 hr⁻¹ or at least 20 hr⁻¹ or at least 30 hr⁻¹ and/or 250 hr⁻¹ or less or 100 hr⁻¹ or less. The total pressure can be from 1 atm (100 kPag) to 200 atm (20.2 MPag), and preferably 100 atm (10.1 MPag) or less. Optionally, the oligomerization feed can be exposed to the catalyst at a hydrogen partial pressure that is at least 1% of the total pressure, such as at least 5% of the total pressure or at least 10% of the total pressure. The reaction products can then be fractionated to separate light ends (including unreacted ethylene) from any naphtha, distillate fuel, and lubricant or wax products. In a single step process, a higher proportion of fuel products are formed.

In a two step process, a first oligomerization to C₆+ olefins (i.e., C₆ and higher carbon number oligomers) is achieved under gas phase conditions similar to those described above. The product is then condensed to recover the C₆+ olefins and preferably to recover C₁₀+ olefins (i.e., C₁₀ and higher carbon number oligomers). Recovering the C₆+ olefins roughly corresponds to recovering an intermediate portion from the first oligomerization having a boiling point of 60°C or greater. Recovering the C₁₀+ olefins roughly corresponds to recovering an intermediate portion with a boiling point of at least 170°C. Thus, the intermediate product that is recovered for further oligomerization can correspond to an intermediate product with an initial boiling point of at least 60°C, such as at least 100°C, or at least 150°C. The resulting intermediate product is then further oligomerized in presence of an acid catalyst under liquid phase conditions to produce higher molecular weight molecules, preferably with more than 20 carbons atoms (C₂₀+), and more preferably with more than 26 carbon atoms (C₂₆+). For the liquid phase oligomerization, the olefin-containing feed from the first stage can be exposed to the catalyst at a temperature from 20°C to 300°C, such as at least 25°C or at least 50°C or at least 100°C and/or preferably 250°C or less, more preferably 225°C or less. The total pressure can be from 1 atm (100 kPag) to 200 atm (20.2 MPag), and preferably 100 atm (10.1 MPag) or less. Optionally, the liquid oligomerization feed can be exposed to the catalyst at a hydrogen partial pressure that is at least 1% of the total pressure, such as at least 5% of the total pressure or at least 10% of the total pressure.

In some optional embodiments, the oligomerization reaction system can include a recycle loop to allow lower molecular weight oligomers to be recycled to a steam cracker or another type of reaction stage suitable for cracking molecules to form ethylene. For example, in a single step process, the oligomers formed during oligomerization can be roughly divided into three types of compounds. A first type of compounds corresponds to lubricant boiling range compounds. Such compounds often contain at least 25 or 26 carbon atoms. A second group of compounds corresponds to distillate fuel compounds, such as diesel and/or kerosene. These compounds tend to have between 10 to 25 carbon atoms. A third type of compound corresponds to compounds having 10 carbon atoms or less. Such compounds correspond to naphtha boiling range compounds. Due to the lower value of naphtha relative to lubricant base oils, a portion of the naphtha boiling range compounds can optionally be recycled to a steam cracker in order to make additional dilute ethylene feedstock. This can allow the naphtha boiling range compounds to be recycled and converted into higher value products. Optionally, a portion of the distillate fuels can also be recycled to a steam cracker, or the distillate fuels portion can be used as a product stream.

Due to the presence of H₂S, organic sulfur, and/or other sulfur compounds in refinery FCC off-gas, the resulting oligomers also include organic sulfur. The amount of organic sulfur in the oligomers generated from the dilute ethylene feed is at least 25 wppm, for example at least 50 wppm, or at least 100 wppm, or at least 200 wppm, or at least 400 wppm. A subsequent hydrotreating process can be used to remove this sulfur from the oligomers.

### Hydroprocessing for Production of Distillate Fuels and Lubricant Basestocks

After forming compounds by oligomerization, the oligomers can be hydroprocessed to remove contaminants and/or improve product properties, such as cold flow properties. Hydrotreatment (or mild hydrocracking) can be used for removal of contaminants, and optionally to provide some viscosity index uplift, while catalytic dewaxing (by isomerization or cracking) can be used to improve cold flow properties.

In the discussion below, a stage can correspond to a single reactor or a plurality of reactors. Optionally, multiple parallel reactors can be used to perform one or more of the processes, or multiple parallel reactors can be used for all processes in a stage. Each stage and/or reactor can include one or more catalyst beds containing hydroprocessing catalyst. Note that a "bed" of catalyst in the discussion below can refer to a partial physical catalyst bed. For example, a catalyst bed within a reactor could be filled partially with a hydrocracking catalyst and partially with a dewaxing catalyst. For convenience in description, even though the two catalysts may be stacked together in a single catalyst bed, the hydrocracking catalyst and dewaxing catalyst can each be referred to conceptually as separate catalyst beds.

In the discussion herein, reference will be made to a hydroprocessing reaction system. The hydroprocessing reaction system corresponds to the one or more stages, such as two stages and/or reactors and an optional intermediate separator, that are used to expose a feed to a plurality of catalysts under hydroprocessing conditions. The plurality of catalysts can be distributed between the stages and/or reactors in any convenient manner, with some preferred methods of arranging the catalyst described herein.

Various types of hydroprocessing can be used in the production of fuels and/or lubricant base oils. Typical processes include a catalytic dewaxing and/or hydrocracking process to modify viscosity properties and/or cold flow properties, such as pour point or cloud point. The hydrocracked and/or dewaxed feed can then be hydrofinished, for example, to saturate olefins and aromatics from the product. In addition to the above, a hydrotreatment stage can also be used for contaminant removal. The hydrotreatment of the oligomer products can be performed prior to or after the hydrocracking and/or dewaxing.

### Hydrotreatment Conditions

Hydrotreatment is typically used to reduce the sulfur, nitrogen, and aromatic content of a feed. The catalysts used for hydrotreatment of the heavy portion of the crude oil from the flash separator can include conventional hydroprocessing catalysts, such as those that comprise at least one Group VIII non-noble metal (Columns 8 - 10 of IUPAC periodic table), preferably Fe, Co, and/or Ni, such as Co and/or Ni; and at least one Group VI metal (Column 6 of IUPAC periodic table), preferably Mo and/or W. Such hydroprocessing catalysts optionally include transition metal sulfides that are impregnated or dispersed on a refractory support or carrier such as alumina and/or silica. The support or carrier itself typically has no significant/measurable catalytic activity. Substantially carrier- or support-free catalysts, commonly referred to as bulk catalysts, generally have higher volumetric activities than their supported counterparts.

The catalysts can either be in bulk form or in supported form. In addition to alumina and/or silica, other suitable support/carrier materials can include, but are not limited to, zeolites, titania, silica-titania, and titania-alumina. Suitable aluminas are porous aluminas such as gamma or eta having average pore sizes from 50 to 200 Å, or 75 to 150 Å; a surface area from 100 to 300 m²/g, or 150 to 250 m²/g; and a pore volume of from 0.25 to 1.0 cm³/g, or 0.35 to 0.8 cm³/g. More generally, any convenient size, shape, and/or pore size distribution for a catalyst suitable for hydrotreatment of a distillate (including lubricant base oil) boiling range feed in a conventional manner may be used. It is within the scope of the present disclosure that more than one type of hydroprocessing catalyst can be used in one or multiple reaction vessels.

The at least one Group VIII non-noble metal, in oxide form, can typically be present in an amount ranging from 2 wt% to 40 wt%, preferably from 4 wt% to 15 wt%. The at least one Group VI metal, in oxide form, can typically be present in an amount ranging from 2 wt% to 70 wt%, preferably for supported catalysts from 6 wt% to 40 wt% or from 10 wt% to 30 wt%. These weight percents are based on the total weight of the catalyst. Suitable metal catalysts include cobalt/molybdenum (1-10% Co as oxide, 10-40% Mo as oxide), nickel/molybdenum (1-10% Ni as oxide, 10-40% Co as oxide), or nickel/tungsten (1-10% Ni as oxide, 10-40% W as oxide) on alumina, silica, silica-alumina, or titania.

The hydrotreatment is carried out in the presence of hydrogen. A hydrogen stream is, therefore, fed or injected into a vessel or reaction zone or hydroprocessing zone in which the hydroprocessing catalyst is located. Hydrogen, which is contained in a hydrogen "treat gas," is provided to the reaction zone. Treat gas, as referred to in this disclosure, can be either pure hydrogen or a hydrogen-containing gas, which is a gas stream containing hydrogen in an amount that is sufficient for the intended reaction(s), optionally including one or more other gasses (e.g., nitrogen and light hydrocarbons such as methane), and which will not adversely interfere with or affect either the reactions or the products. Impurities, such as H₂S and NH₃ are undesirable and would typically be removed from the treat gas before it is conducted to the reactor. The treat gas stream introduced into a reaction stage will preferably contain at least 50 vol. % and more preferably at least 75 vol. % hydrogen.

Hydrotreating conditions include temperatures of 200°C to 450°C, or 315°C to 425°C; pressures of 250 psig (1.8 MPag) to 5000 psig (34.6 MPag) or 300 psig (2.1 MPag) to 3000 psig (20.8 MPag); liquid hourly space velocities (LHSV) of 0.1 hr⁻¹ to 10 hr⁻¹; and hydrogen treat rates of 200 scf/B (35.6 m³/m³) to 10,000 scf/B (1781 m³/m³), or 500 (89 m³/m³) to 10,000 scf/B (1781 m³/m³).

### Catalytic Dewaxing Process

Suitable dewaxing catalysts can include molecular sieves such as crystalline aluminosilicates (zeolites). In an embodiment, the molecular sieve can comprise, consist essentially of, or be ZSM-5, ZSM-22, ZSM-23, ZSM-35, ZSM-48, zeolite Beta, or a combination thereof, for example ZSM-23 and/or ZSM-48, or ZSM-48 and/or zeolite Beta. Optionally but preferably, molecular sieves that are selective for dewaxing by isomerization as opposed to cracking can be used, such as ZSM-48, zeolite Beta, ZSM-23, or a combination thereof. Additionally or alternately, the molecular sieve can comprise, consist essentially of, or be a 10-member ring 1-D molecular sieve. Examples include EU-1, ZSM-35 (or ferrierite), ZSM-11, ZSM-57, NU-87, SAPO-11, ZSM-48, ZSM-23, and ZSM-22. Preferred materials are EU-2, EU-11, ZBM-30, ZSM-48, or ZSM-23. ZSM-48 is most preferred. Note that a zeolite having the ZSM-23 structure with a silica to alumina ratio of from 20:1 to 40:1 can sometimes be referred to as SSZ-32. Other molecular sieves that are isostructural with the above materials include Theta-1, NU-10, EU-13, KZ-1, and NU-23. Optionally but preferably, the dewaxing catalyst can include a binder for the molecular sieve, such as alumina, titania, silica, silica-alumina, zirconia, or a combination thereof, for example alumina and/or titania or silica and/or zirconia and/or titania.

Preferably, the dewaxing catalysts used in processes according to the disclosure are catalysts with a low ratio of silica to alumina. For example, for ZSM-48, the ratio of silica to alumina in the zeolite can be less than 200:1, such as less than 110:1, or less than 100:1, or less than 90:1, or less than 75:1. In various embodiments, the ratio of silica to alumina can be from 50:1 to 200:1, such as 60:1 to 160:1, or 70:1 to 100:1.

In various embodiments, the catalysts according to the disclosure further include a metal hydrogenation component. The metal hydrogenation component is typically a Group VI and/or a Group VIII metal. Preferably, the metal hydrogenation component is a Group VIII noble metal. Preferably, the metal hydrogenation component is Pt, Pd, or a mixture thereof. In an alternative preferred embodiment, the metal hydrogenation component can be a combination of a non-noble Group VIII metal with a Group VI metal. Suitable combinations can include Ni, Co, or Fe with Mo or W, preferably Ni with Mo or W.

The metal hydrogenation component may be added to the catalyst in any convenient manner. One technique for adding the metal hydrogenation component is by incipient wetness. For example, after combining a zeolite and a binder, the combined zeolite and binder can be extruded into catalyst particles. These catalyst particles can then be exposed to a solution containing a suitable metal precursor. Alternatively, metal can be added to the catalyst by ion exchange, where a metal precursor is added to a mixture of zeolite (or zeolite and binder) prior to extrusion.

The amount of metal in the catalyst can be at least 0.1 wt% based on catalyst, or at least 0.15 wt% or at least 0.2 wt%, or at least 0.25 wt%, or at least 0.3 wt%, or at least 0.5 wt% based on catalyst. The amount of metal in the catalyst can be 20 wt% or less based on catalyst, or 10 wt% or less, or 5 wt% or less, or 2.5 wt% or less, or 1 wt% or less. For embodiments where the metal is Pt, Pd, another Group VIII noble metal, or a combination thereof, the amount of metal can be from 0.1 to 5 wt%, preferably from 0.1 to 2 wt%, or 0.25 to 1.8 wt%, or 0.4 to 1.5 wt%. For embodiments where the metal is a combination of a non-noble Group VIII metal with a Group VI metal, the combined amount of metal can be from 0.5 wt% to 20 wt%, or 1 wt% to 15 wt%, or 2.5 wt% to 10 wt%.

The dewaxing catalysts useful in processes according to the disclosure can also include a binder. In some embodiments, the dewaxing catalysts used in process according to the disclosure are formulated using a low surface area binder, a low surface area binder represents a binder with a surface area of 100 m²/g or less, or 80 m²/g or less, or 70 m²/g or less. The amount of zeolite in a catalyst formulated using a binder can be from 30 wt% zeolite to 90 wt% zeolite relative to the combined weight of binder and zeolite. Preferably, the amount of zeolite is at least 50 wt% of the combined weight of zeolite and binder, such as at least 60 wt% or from 65 wt% to 80 wt%.

A zeolite can be combined with binder in any convenient manner. For example, a bound catalyst can be produced by starting with powders of both the zeolite and binder, combining and mulling the powders with added water to form a mixture, and then extruding the mixture to produce a bound catalyst of a desired size. Extrusion aids can also be used to modify the extrusion flow properties of the zeolite and binder mixture. The amount of framework alumina in the catalyst may range from 0.1 to 3.33 wt%, or 0. 1 to 2.7 wt%, or 0.2 to 2 wt%, or 0.3 to 1 wt%.

Process conditions in a catalytic dewaxing zone in a sour environment can include a temperature of from 200 to 450°C, preferably 270 to 400°C, a hydrogen partial pressure of from 1.8 MPag to 34.6 MPag (250 psig to 5000 psig), preferably 4.8 MPag to 20.8 MPag, and a hydrogen circulation rate of from 35.6 m³/m³ (200 SCF/B) to 1781 m³/m³ (10,000 scf/B), preferably 178 m³/m³ (1000 SCF/B) to 890.6 m³/m³ (5000 SCF/B). In still other embodiments, the conditions can include temperatures in the range of 600°F (343°C) to 815°F (435°C), hydrogen partial pressures of from 500 psig to 3000 psig (3.5 MPag-20.9 MPag), and hydrogen treat gas rates of from 213 m³/m³ to 1068 m³/m³ (1200 SCF/B to 6000 SCF/B). These latter conditions may be suitable, for example, if the dewaxing stage is operating under sour conditions. The liquid hourly space velocity can vary depending on the relative amount of hydrocracking catalyst used versus dewaxing catalyst. Relative to the combined amount of hydrocracking and dewaxing catalyst, the LHSV can be from 0.2 h⁻¹ to 10 h⁻¹, such as from 0.5 h⁻¹ to 5 h⁻¹ and/or from 1 h⁻¹ to 4 h⁻¹. Depending on the relative amount of hydrocracking catalyst and dewaxing catalyst used, the LHSV relative to only the dewaxing catalyst can be from 0.25 h⁻¹ to 50 h⁻¹, such as from 0.5 h⁻¹ to 20 h⁻¹, and preferably from 1.0 h⁻¹ to 4.0 h⁻1.

### Hydrocracking Conditions

Hydrocracking catalysts typically contain sulfided base metals on acidic supports, such as amorphous silica alumina, cracking zeolites such as USY, or acidified alumina. Often these acidic supports are mixed or bound with other metal oxides such as alumina, titania or silica. Examples of suitable acidic supports include acidic molecular sieves, such as zeolites or silicoaluminophophates. One example of suitable zeolite is USY, such as a USY zeolite with cell size of 24.25 Angstroms or less. Additionally or alternately, the catalyst can be a low acidity molecular sieve, such as a USY zeolite with a Si to Al ratio of at least 20, and preferably at least 40 or 50. Zeolite Beta is another example of a potentially suitable hydrocracking catalyst. Non-limiting examples of metals for hydrocracking catalysts include metals or combinations of metals that include at least one Group VIII metal, such as nickel, nickel-cobalt-molybdenum, cobalt-molybdenum, nickel-tungsten, nickel-molybdenum, and/or nickel-molybdenum-tungsten. Additionally or alternately, hydrocracking catalysts with noble metals can also be used. Non-limiting examples of noble metal catalysts include those based on platinum and/or palladium. Support materials which may be used for both the noble and non-noble metal catalysts can comprise a refractory oxide material such as alumina, silica, alumina-silica, kieselguhr, diatomaceous earth, magnesia, zirconia, or combinations thereof, with alumina, silica, alumina-silica being the most common (and preferred, in one embodiment).

In various aspects, the conditions selected for hydrocracking can depend on the desired level of conversion, the level of contaminants in the input feed to the hydrocracking stage, and potentially other factors. For example, a hydrocracking process performed prior to hydrotreatment can be carried out at temperatures of 550°F (288°C) to 840°F (449°C), hydrogen partial pressures of from 250 psig to 5000 psig (1.8 MPag to 34.6 MPag), liquid hourly space velocities of from 0.05 h⁻¹ to 10 h⁻¹, and hydrogen treat gas rates of from 35.6 m³/m³ to 1781 m³/m³ (200 SCF/B to 10,000 SCF/B). In other embodiments, the conditions can include temperatures in the range of 600°F (343°C) to 815°F (435°C), hydrogen partial pressures of from 500 psig to 3000 psig (3.5 MPag-20.9 MPag), and hydrogen treat gas rates of from 213 m³/m³ to 1068 m³/m³ (1200 SCF/B to 6000 SCF/B). The LHSV relative to only the hydrocracking catalyst can be from 0.25 h⁻¹ to 50 h⁻¹, such as from 0.5 h⁻¹ to 20 h⁻¹, and preferably from 1.0 h⁻¹ to 4.0 h⁻¹. Alternatively, milder hydrocracking conditions can also be desirable, such as for a hydrocracking stage located downstream from the hydrotreating stage. In such an alternative, suitable hydrocracking conditions can include temperatures of 550°F (288°C) to 840°F (449°C), hydrogen partial pressures of from 250 psig to 5000 psig (1.8 MPag to 34.6 MPag), liquid hourly space velocities of from 0.05 h⁻¹ to 10 h⁻¹, and hydrogen treat gas rates of from 35.6 m³/m³ to 1781 m³/m³ (200 SCF/B to 10,000 SCF/B). In other embodiments, the conditions can include temperatures in the range of 600°F (343°C) to 815°F (435°C), hydrogen partial pressures of from 500 psig to 3000 psig (3.5 MPag-20.9 MPag), and hydrogen treat gas rates of from 213 m³/m³ to 1068 m³/m³ (1200 SCF/B to 6000 SCF/B).

### Hydrofinishing and/or Aromatic Saturation Process

In some aspects, a hydrofinishing and/or aromatic saturation stage can also be provided. The hydrofinishing and/or aromatic saturation can occur after the last hydrocracking or dewaxing stage. The hydrofinishing and/or aromatic saturation can occur either before or after fractionation. If hydrofinishing and/or aromatic saturation occurs after fractionation, the hydrofinishing can be performed on one or more portions of the fractionated product, such as being performed on the bottoms from the reaction stage (i.e., the hydrocracker bottoms). Alternatively, the entire effluent from the last hydrocracking or dewaxing process can be hydrofinished and/or undergo aromatic saturation.

In some situations, a hydrofinishing process and an aromatic saturation process can refer to a single process performed using the same catalyst. Alternatively, one type of catalyst or catalyst system can be provided to perform aromatic saturation, while a second catalyst or catalyst system can be used for hydrofinishing. Typically a hydrofinishing and/or aromatic saturation process will be performed in a separate reactor from dewaxing or hydrocracking processes for practical reasons, such as facilitating use of a lower temperature for the hydrofinishing or aromatic saturation process. However, an additional hydrofinishing reactor following a hydrocracking or dewaxing process but prior to fractionation could still be considered part of a second stage of a reaction system conceptually.

Hydrofinishing and/or aromatic saturation catalysts can include catalysts containing Group VI metals, Group VIII metals, and mixtures thereof. In an embodiment, preferred metals include at least one metal sulfide having a strong hydrogenation function. In another embodiment, the hydrofinishing catalyst can include a Group VIII noble metal, such as Pt, Pd, or a combination thereof. The mixture of metals may also be present as bulk metal catalysts wherein the amount of metal is 30 wt. % or greater based on catalyst. Suitable metal oxide supports include low acidic oxides such as silica, alumina, silica-aluminas or titania, preferably alumina. The preferred hydrofinishing catalysts for aromatic saturation will comprise at least one metal having relatively strong hydrogenation function on a porous support. Typical support materials include amorphous or crystalline oxide materials such as alumina, silica, and silica-alumina. The support materials may also be modified, such as by halogenation, or in particular fluorination. The metal content of the catalyst is often as high as 20 weight percent for non-noble metals. In an embodiment, a preferred hydrofinishing catalyst can include a crystalline material belonging to the M41S class or family of catalysts. The M41S family of catalysts are mesoporous materials having high silica content. Examples include MCM-41, MCM-48 and MCM-50. A preferred member of this class is MCM-41. If separate catalysts are used for aromatic saturation and hydrofinishing, an aromatic saturation catalyst can be selected based on activity and/or selectivity for aromatic saturation, while a hydrofinishing catalyst can be selected based on activity for improving product specifications, such as product color and polynuclear aromatic reduction.

Hydrofinishing conditions can include temperatures from 125°C to 425°C, preferably 180°C to 280°C, a hydrogen partial pressure from 500 psig (3.4 MPa) to 3000 psig (20.7 MPa), preferably 1500 psig (10.3 MPa) to 2500 psig (17.2 MPa), and liquid hourly space velocity from 0.1 hr⁻¹ to 5 hr⁻¹ LHSV, preferably 0.5 hr⁻¹ to 1.5 hr⁻¹. Additionally, a hydrogen treat gas rate of from 35.6 m³/m³ to 1781 m³/m³ (200 SCF/B to 10,000 SCF/B) can be used.

After hydroprocessing, the hydroprocessed oligomers can be fractionated to form at least one naphtha fraction, at least one diesel or distillate fuel fraction, and at least one lubricant base oil fraction. Optionally, a fractionation can also be performed on the oligomer stream prior to hydroprocessing, so that a portion of the lower boiling (naphtha) compounds can be recycled for cracking to form more dilute ethylene.

### Example of Configuration for Integrated Reaction System

FIG. 1 shows a schematic example of configuration for oligomerizing a dilute ethylene feed to form fuels and lubricant base oils. In the embodiment shown in FIG. 1, a gas feed 102 containing ethylene and other impurities and/or diluents can be used as the initial feed for oligomerization. Gas feed 102 is an off-gas from an FCC process, optionally after removal of C₃ and C₄ components. The input feeds for steam cracker 110 can correspond to a separate 104 of larger molecules and/or recycled oligomers (and possibly ethylene) 164 from the oligomerization process. Feed 102 is then passed into an oligomerization stage 120. In FIG. 1, the oligomerization stage 120 is shown as a single reactor, but a two-stage reactor and/or multiple reactors can also be used. The oligomerization stage 120 generates an oligomerized output stream 125. Output stream 125 can then optionally be fractionated to form a lower boiling portion 132, an intermediate boiling portion 134, and a higher boiling portion 136. For example, lower boiling portion 132 can correspond roughly to compounds containing 10 carbon atoms or less. This includes unreacted ethylene, other light ends, and naphtha boiling range compounds. The lower boiling portion 132 can be used as part of a recycle feed 164 for steam cracker 110, or the lower boiling portion can be used as a naphtha product 162, typically after additional separation to remove compounds with a boiling point of less than 100°F (i.e., roughly compounds with less than 5 carbon atoms). The intermediate boiling portion 134 is optional, and can be included as part of higher boiling portion 136. In the configuration shown in FIG. 1, the intermediate boiling portion does not undergo further hydroprocessing. Instead, the intermediate boiling portion can exit the reaction system for use as a diesel fuel, possibly after additional processing. The intermediate boiling portion can correspond to compounds, for example, with 10 to 25 carbon atoms, such as compounds with 10 to 20 carbon atoms. The higher boiling portion 136 contains the remaining portion of the oligomerized output stream, which can correspond to compounds with 20 or more carbon atoms, such as 25 or more carbon atoms.

The higher boiling portion 136 is then passed into one or more hydroprocessing stage(s) 140 for contaminant removal, and improvement of viscosity and/or cold flow properties. Hydroprocessing stage(s) can include one or more hydrotreatment stages for contaminant removal and one or more dewaxing and/or hydrocracking stages for property improvements. The severity of the hydroprocessing stages can vary, depending on the amount of property improvement needed to produce a desirable lubricant base oil, such as a Group II, Group II+, or Group III base oil. The hydroprocessed effluent 145 can then be fractionated to generate at least one lower boiling portion 152 (including light ends and naphtha), at least one intermediate boiling portion 154 (diesel and/or kerosene), and at least one higher boiling portion 156 (lubricant base oil). The at least one lubricant base oil portion 156 preferably corresponds to a Group II, Group II+, and/or Group III lubricant base oil portion.

## Claims

1. A method for forming fuel and lubricant products, comprising:
exposing a feedstock comprising 5 to 50 vol% of ethylene to a catalyst comprising a zeolite with 10-member rings under effective oligomerization conditions to produce an oligomerized effluent, wherein the feedstock consists of an off-gas from a fluid catalytic cracking process, wherein the off-gas comprises H₂S, organic sulfur, and/or other sulfur compounds, and wherein the oligomerized effluent comprises organic sulfur in an amount of at least 25 wppm;
hydroprocessing at least a portion of the oligomerized effluent under effective hydroprocessing conditions to form a hydroprocessed effluent, wherein hydroprocessing at least a portion of the oligomerized effluent comprises hydrotreating at least a portion of the oligomerized effluent under hydrotreating conditions including temperatures of 200°C to 450°C, pressures of 250 psig (1.8 MPag) to 5000 psig (34.6 MPag), liquid hourly space velocities (LHSV) of 0.1 hr⁻¹ to 10 hr⁻¹, and hydrogen treat rates of 200 scf/B (35.6 m³/m³) to 10,000 scf/B (1781 m³/m³); and
separating the hydroprocessed effluent to form a fraction having a boiling point of 350°F (177°C) or less, one or more lubricant base oil fractions, and at least one diesel fuel fraction having a lower boiling range than at least one lubricant base oil fraction.

2. The method of claim 1, wherein exposing the feedstock comprising 5 to 50 vol% of ethylene to a catalyst comprising a zeolite with 10-member rings under effective oligomerization conditions to produce an oligomerized effluent comprises:
exposing the feedstock to a first catalyst comprising a zeolite with 10-member rings under effective gas phase oligomerization conditions to form an intermediate oligomerized product;
separating the intermediate oligomerized product to form at least a gas phase intermediate product and an intermediate product with an initial boiling point of at least 60°C, preferably of at least 150 °C; and
exposing at least a portion of the intermediate product with a boiling point of at least 60°C to a second catalyst comprising a zeolite with 10-member rings under effective liquid phase oligomerization conditions to form the oligomerized effluent.

3. The method of claim 2, further comprising:
recycling at least a portion of the gas phase intermediate product to a cracking process;
cracking the recycled fraction to form an ethylene-containing effluent; and
exposing the ethylene-containing effluent to the catalyst comprising a zeolite with 10-member rings under the effective oligomerization conditions.

4. The method of claim 1, wherein hydroprocessing the oligomerized effluent comprises hydrotreating the oligomerized effluent, catalytically dewaxing the oligomerized effluent, hydrocracking the oligomerized effluent, hydrofinishing the oligomerized effluent, or a combination thereof.

5. The method of claim 4, wherein hydroprocessing the oligomerized effluent comprises hydrotreating the oligomerized effluent to form a hydrotreated effluent, and catalytically dewaxing the hydrotreated effluent.

6. The method of claim 4, wherein hydroprocessing the oligomerized effluent comprises hydrocracking the oligomerized effluent to form a hydrocracked effluent, and hydrotreating the hydrocracked effluent.

7. The method of claim 1, wherein at least a portion of the C₃ and C₄ compounds in the off-gas from the fluid catalytic cracking process are separated from the feedstock prior to exposing the feedstock to the catalyst comprising the zeolite having 10-member rings.

8. The method of claim 1, further comprising:
recycling at least a portion of the fraction having a boiling point of 350°F (177°C) or less to a cracking process;
cracking the recycled fraction to form an ethylene-containing effluent; and
exposing the ethylene-containing effluent to the catalyst comprising a zeolite with 10-member rings under the effective oligomerization conditions.

9. The method of claim 1, wherein the catalyst comprising a zeolite with 10-member rings comprises ZSM-5, ZSM-22, ZSM-23, ZSM-48, or a combination thereof, the zeolite preferably having a silica to alumina ratio of at least 40.

10. The method of claim 1, wherein the catalyst comprising a zeolite with 10-member rings further comprises at least one of a metal hydrogenation component and a binder.

11. The method of claim 1, wherein hydroprocessing the oligomerized effluent under effective hydroprocessing conditions to form a hydroprocessed effluent comprises hydroprocessing a portion of the oligomerized effluent having a T5 boiling point of at least 177°C (350°F), preferably at least 316°C (600 °F).

12. The method of claim 1, wherein at least one of the one or more lubricant base oil fractions comprises a Group II, Group II+, or Group III lubricant base oil.

13. The method of claim 1, wherein the at least a portion of the oligomerized effluent has a sulfur content of at least 50 wppm.

14. The method of claim 1, further comprising subjecting the one or more lubricant base oil fractions and/or at least one diesel fuel fraction to one or more further converting steps selected from the group consisting of hydrocracking, fluid catalytic cracking, isomerizing and combinations thereof.

## Patentansprüche

1. Verfahren zur Bildung von Kraftstoff- und Schmiermittelprodukten, umfassend:
Aussetzen eines Einsatzmaterials, das 5 bis 50 Vol.-% Ethylen umfasst, an einen Katalysator, der einen Zeolith mit 10-gliedrigen Ringen umfasst, unter wirksamen Oligomerisierungsbedingungen, um einen oligomerisierten Abstrom zu erzeugen, wobei das Einsatzmaterial aus einem Abgas aus dem katalytischen Fluidcrackverfahren besteht, wobei das Abgas H₂S, organischen Schwefel und/oder andere Schwefelverbindungen in einer Menge von mindestens 25 Gew.-ppm umfasst;
Hydroverarbeitung von mindestens einem Teil des oligomerisierten Abstroms unter wirksamen Hydroverarbeitungsbedingungen zur Bildung eines hydroverarbeiteten Abstroms, wobei die Hydroverarbeitung von mindestens einem Teil des oligomerisierten Abstroms die Hydrobehandlung mindestens eines Teils des oligomerisierten Abstroms unter Hydroverarbeitungsbedingungen umfasst, die Temperaturen von 200°C bis 450°C, Drücke von 250 psi-Überdruck (1,8 MPa-Überdruck) bis 5000 psi-Überdruck (34,6 MPa-Überdruck), stündliche Flüssigkeitsraumgeschwindigkeiten (LHSV) von 0,1 hr⁻¹ bis 10 hr⁻¹, und Hydrobehandlungsraten von 200 scf/B (35,6 m³/m³) bis 10.000 scf/B (1781 m³/m³) einschließen; und
Trennen des wasseraufbereiteten Abstroms, um eine Fraktion mit einem Siedepunkt von 350°F (177°C) oder weniger, eine oder mehrere Schmiermittelgrundölfraktionen und mindestens eine Dieselkraftstofffraktion mit einem niedrigeren Siedebereich als mindestens eine Schmiermittelgrundölfraktion zu bilden.

2. Verfahren nach Anspruch 1, wobei das Aussetzen des Ausgangsmaterials, das 5 bis 50 Vol.-% Ethylen umfasst, an einen Katalysator, der einen Zeolith mit 10-gliedrigen Ringen umfasst, unter wirksamen Oligomerisierungsbedingungen, um einen oligomerisierten Abstrom zu erzeugen, Folgendes umfasst:
Aussetzen des Ausgangsmaterials an einen ersten Katalysator, der einen Zeolith mit 10-gliedrigen Ringen umfasst, unter wirksamen Gasphasen-Oligomerisierungsbedingungen zur Bildung eines oligomerisierten Zwischenprodukts;
Abtrennen des oligomerisierten Zwischenprodukts zur Bildung eines Gasphasen-Zwischenprodukts und eines Zwischenprodukts mit einem Anfangssiedepunkt von mindestens 60°C, vorzugsweise mindestens 150°C; und
Aussetzen mindestens eines Teils des Zwischenprodukts mit einem Siedepunkt von mindestens 60°C an einen zweiten Katalysator, der einen Zeolith mit 10-gliedrigen Ringen umfasst, unter wirksamen Flüssigphasen-Oligomerisierungsbedingungen, zur Bildung des oligomerisierten Abstroms.

3. Verfahren nach Anspruch 2, ferner umfassend:
Rückführen mindestens eines Teils des Gasphasen-Zwischenprodukts zu einem Crackprozess;
Cracken der rückgeführten Fraktion zur Bildung eines ethylenhaltigen Abstroms; und
Aussetzen des ethylenhaltigen Abstroms dem Katalysator, der einen Zeolith mit 10-gliedrigen Ringen umfasst, unter den wirksamen Oligomerisierungsbedingungen.

4. Verfahren nach Anspruch 1, wobei das Hydroverarbeiten des oligomerisierten Abstroms das Hydrobehandeln des oligomerisierten Abstroms, das katalytische Entwachsen des oligomerisierten Abstroms, das Hydrocracken des oligomerisierten Abstroms, das Hydrofinishing des oligomerisierten Abstroms oder eine Kombination davon umfasst.

5. Verfahren nach Anspruch 4, wobei das Hydroverarbeiten des oligomerisierten Abstroms das Hydrobehandeln des oligomerisierten Abstroms zur Bildung eines hydrobehandelten Abstroms und das katalytische Entwachsen des hydrobehandelten Abstroms umfasst.

6. Verfahren nach Anspruch 4, wobei das Hydroverarbeiten des oligomerisierten Abstroms das Hydrocracken des oligomerisierten Abwassers zur Bildung eines hydrogecrackten Abstroms und die Hydrobehandlung des hydrogecrackten Abstroms umfasst.

7. Verfahren nach Anspruch 1, wobei mindestens ein Teil der C₃- und C₄-Verbindungen in dem Abgas aus dem katalytischen Fluidcrackverfahren von dem Einsatzmaterial abgetrennt wird, bevor das Einsatzmaterial dem Katalysator ausgesetzt wird, der den Zeolith mit 10-gliedrigen Ringen umfasst.

8. Verfahren nach Anspruch 1, ferner umfassend:
Rückführen mindestens eines Teils der Fraktion mit einem Siedepunkt von (350°F) 177°C oder weniger in einen Crackprozess;
Cracken der rückgeführten Fraktion unter Bildung eines Ethylen enthaltenden Abstroms; und
Aussetzen des ethylenhaltigen Abstroms dem Katalysator, der einen Zeolith mit 10-gliedrigen Ringen umfasst, unter den wirksamen Oligomerisierungsbedingungen.

9. Verfahren nach Anspruch 1, wobei der Katalysator, der einen Zeolith mit 10-gliedrigen Ringen umfasst, ZSM-5, ZSM-22, ZSM-23, ZSM-48 oder eine Kombination davon umfasst, wobei der Zeolith vorzugsweise ein Siliziumoxidzu-Aluminiumoxid-Verhältnis von mindestens 40 aufweist.

10. Verfahren nach Anspruch 1, wobei der Katalysator, der einen Zeolith mit 10-gliedrigen Ringen umfasst, ferner mindestens eines von einer Metallhydrierungskomponente und einem Bindemittel umfasst.

11. Verfahren nach Anspruch 1, wobei das Hydroverarbeiten des oligomerisierten Abstroms unter wirksamen Hydroverarbeitungsbedingungen zur Bildung eines hydroverarbeiteten Abstroms das Hydrobehandeln eines Teils des oligo-merisierten Abstroms mit einem T5-Siedepunkt von mindestens 177°C (350°F), vorzugsweise mindestens 316°C (600°F) umfasst.

12. Verfahren nach Anspruch 1, wobei mindestens eine der einen oder mehreren Schmiermittelgrundölfraktionen ein Schmiermittelgrundöl der Gruppe II, der Gruppe II+ oder der Gruppe III umfasst.

13. Verfahren nach Anspruch 1, wobei der mindestens eine Teil des oligomerisierten Abstroms einen Schwefelgehalt von mindestens 50 Gew.ppm aufweist.

14. Verfahren nach Anspruch 1, ferner umfassend das Unterwerfen der einen oder mehreren Schmiermittelgrundölfraktionen und/oder mindestens einer Dieselkraftstofffraktion einem oder mehreren weiteren Umwandlungsschritten, ausgewählt aus der Gruppe, bestehend aus Hydrocracken, fluidkatalytischem Cracken, Isomerisieren und Kombinationen davon.

## Revendications

1. Procédé de formation de carburant et de produits lubrifiants, comprenant les étapes consistant à :
exposer une matière première comprenant 5 à 50 % en volume d'éthylène à un catalyseur comprenant une zéolithe avec des cycles à 10 chaînons dans des conditions d'oligomérisation efficaces pour obtenir un effluent oligomérisé, où la matière première est constituée d'effluents gazeux issus d'un procédé de craquage catalytique fluide, où les effluents gazeux comprennent H₂S, du soufre organique et/ou d'autres composés soufrés, et où l'effluent oligomérisé comprend du soufre organique à une teneur d'au moins 25 ppm en masse ;
hydrotraiter au moins une partie de l'effluent oligomérisé dans des conditions d'hydrotraitement efficaces pour former un effluent hydrotraité, où l'hydrotraitement d'au moins une partie de l'effluent oligomérisé comprend l'hydrotraitement d'au moins une partie de l'effluent oligomérisé dans des conditions d'hydrotraitement incluant des températures de 200 °C à 450 °C, des pressions de 250 psig (1,8 MPag) à 5000 psig (34,6 MPag), des vitesses volumiques horaires liquides (LHSV) de 0,1 hr⁻¹ à 10 hr⁻¹, et des taux de traitement par hydrogène de 200 scf/B (35,6 m³/m³) à 10 000 scf/B (1781 m³/m³) ; et
séparer l'effluent hydrotraité pour former une fraction ayant un point d'ébullition de 177 °C (350 °F) ou moins, une ou plusieurs fractions d'huile de base lubrifiante, et au moins une fraction de carburant diesel d'intervalle d'ébullition plus faible que celui d'au moins une fraction d'huile de base lubrifiante.

2. Procédé selon la revendication 1, où l'exposition de la matière première comprenant 5 à 50 % en volume d'éthylène à un catalyseur comprenant une zéolithe avec des cycles à 10 chaînons dans des conditions d'oligomérisation efficaces pour obtenir un effluent oligomérisé comprend les étapes consistant à :
exposer la matière première à un premier catalyseur comprenant une zéolithe avec des cycles à 10 chaînons dans des conditions d'oligomérisation en phase gazeuse efficaces pour former un produit oligomérisé intermédiaire ;
séparer le produit oligomérisé intermédiaire pour former au moins un produit intermédiaire en phase gazeuse et un produit intermédiaire de point d'ébullition initial d'au moins 60 °C, préférentiellement d'au moins 150 °C ; et
exposer au moins une partie du produit intermédiaire de point d'ébullition d'au moins 60 °C à un deuxième catalyseur comprenant une zéolithe avec des cycles à 10 chaînons dans des conditions d'oligomérisation en phase liquide efficaces pour former l'effluent oligomérisé.

3. Procédé selon la revendication 2, comprenant en outre les étapes consistant à :
recycler au moins une partie du produit intermédiaire en phase gazeuse vers un processus de craquage ;
craquer la fraction recyclée pour former un effluent contenant de l'éthylène ; et
exposer l'effluent contenant de l'éthylène au catalyseur comprenant une zéolithe avec des cycles à 10 chaînons dans les conditions d'oligomérisation efficaces.

4. Procédé selon la revendication 1, où l'hydrotraitement de l'effluent oligomérisé comprend les étapes consistant à hydrotraiter l'effluent oligomérisé, déparaffiner catalytiquement l'effluent oligomérisé, hydrocraquer l'effluent oligomérisé, hydrofinir l'effluent oligomérisé, ou l'une de leurs combinaisons.

5. Procédé selon la revendication 4, où l'hydrotraitement de l'effluent oligomérisé comprend les étapes consistant à hydrotraiter l'effluent oligomérisé pour former un effluent hydrotraité et déparaffiner catalytiquement l'effluent hydrotraité.

6. Procédé selon la revendication 4, où l'hydrotraitement de l'effluent oligomérisé comprend les étapes consistant à hydrocraquer l'effluent oligomérisé pour former un effluent hydrocraqué et hydrotraiter l'effluent hydrocraqué.

7. Procédé selon la revendication 1, où au moins une partie des composés en C₃ et C₄ dans les effluents gazeux du processus de craquage catalytique fluide est séparée de la matière première avant exposition de la matière première au catalyseur comprenant la zéolithe ayant des cycles à 10 chaînons.

8. Procédé selon la revendication 1, comprenant en outre les étapes consistant à :
recycler au moins une partie de la fraction de point d'ébullition de 177 °C (350 °F) ou moins vers un processus de craquage ;
craquer la fraction recyclée pour former un effluent contenant de l'éthylène ; et
exposer l'effluent contenant de l'éthylène au catalyseur comprenant une zéolithe avec des cycles à 10 chaînons dans les conditions d'oligomérisation efficaces.

9. Procédé selon la revendication 1, où le catalyseur comprenant une zéolithe avec des cycles à 10 chaînons comprend ZSM-5, ZSM-22, ZSM-23, ZSM-48 ou l'une de leurs combinaisons, la zéolithe présentant préférentiellement un rapport silice sur alumine d'au moins 40.

10. Procédé selon la revendication 1, où le catalyseur comprenant une zéolithe avec des cycles à 10 chaînons comprend en outre au moins l'un des membres du groupe constitué par un composant d'hydrogénation métallique et un agent liant.

11. Procédé selon la revendication 1, où l'hydrotraitement de l'effluent oligomérisé dans des conditions d'hydrotraitement efficaces pour former un effluent hydrotraité comprend l'étape consistant à hydrotraiter une partie de l'effluent oligomérisé de point d'ébullition T5 d'au moins 177 °C (350 °F), préférentiellement d'au moins 316 °C (600 °F).

12. Procédé selon la revendication 1, où au moins l'une des une ou plusieurs fractions d'huile de base lubrifiante comprend une huile de base lubrifiante du groupe II, du groupe II+ ou du groupe III.

13. Procédé selon la revendication 1, où l'au moins une partie de l'effluent oligomérisé présente une teneur en soufre d'au moins 50 ppm en masse.

14. Procédé selon la revendication 1, comprenant en outre l'étape consistant à soumettre la ou les fractions d'huile de base lubrifiante et/ou au moins une fraction de carburant diesel à une ou plusieurs étapes de conversion supplémentaires choisies dans le groupe constitué par l'hydrocraquage, le craquage catalytique fluide, l'isomérisation et leurs combinaisons.
